# EUROPÄISCHE PATENTSCHRIFT

(11) **EP 1 973 606 B1**
(45) Veröffentlichungstag und Bekanntmachung des Hinweises auf die Patenterteilung: **17.10.2018**
(21) Anmeldenummer: 06829411.5
(22) Anmeldetag: 08.12.2006
(51) Int. Cl.: A61N 5/10

(54) **VORRICHTUNG ZUM BESTRAHLEN VON TUMORGEWEBE EINES PATIENTEN MIT EINEM TEILCHENSTRAHL**
DEVICE FOR IRRADIATING TUMOROUS TISSUE OF A PATIENT WITH A PARTICLE BEAM
INSTALLATION POUR IRRADIER UN TISSU TUMORAL CHEZ UN PATIENT AU MOYEN D'UN FAISCEAU DE PARTICULES

(30) Priorität: 22.12.2005 DE 102005063220; 14.09.2006 DE 102006043066
(43) Veröffentlichungstag der Anmeldung: 01.10.2008
(73) Patentinhaber: GSI Helmholtzzentrum für Schwerionenforschung GmbH, 64291 Darmstadt (DE)
(72) Erfinder: BERT, Christoph, 63739 Aschaffenburg (DE); RIETZEL, Eike, 64289 Darmstadt (DE); KRAFT, Gerhard, 64291 Darmstadt (DE)
(74) Vertreter: Mergel, Volker
(86) Internationale Anmeldenummer: PCT/EP2006/011799
(87) Internationale Veröffentlichungsnummer: WO 2007/079854

(56) Entgegenhaltungen:
- EP-A- 1 121 957
- EP-A1- 1 880 673
- WO-A-2005/120641
- WO-A2-2007/014110
- DE-A1- 10 031 074
- DE-A1- 19 907 098
- US-A1- 2004 002 641
- LOEF J ET AL: "An adaptive control algorithm for optimization of intensity modulated radiotherapy considering uncertainties in beam profiles, patient set-up and internal organ motion" PHYSICS IN MEDICINE AND BIOLOGY, TAYLOR AND FRANCIS LTD. LONDON, GB, Bd. 43, Juni 1998 (1998-06), Seiten 1605-1628, XP002180223 ISSN: 0031-9155
- PAUL KEALL ED - WAZER DAVID: "4-Dimensional Computed Tomography Imaging and Treatment Planning", SEMINARS IN RADIATION ONCOLOGY, SAUNDERS, PHILADELPHIA, PA, US, vol. 14, no. 1, 1 January 2004 (2004-01-01), pages 81-90, XP002461023, ISSN: 1053-4296, DOI: 10.1053/J.SEMRADONC.2003.10.006

## Beschreibung

Die Erfindung betrifft eine Vorrichtung zur Bestrahlung von von Tumorgewebe eines Patienten, mit einem Teilchenstrahl gemäß des Anspruchs 1. Die Erfindung betrifft außerdem ein Verfahren zur Dosisberechnung für gescannte Teilchenstrahlen für eine Vorrichtung zur Bestrahlung von Tumorgewebe eines Patienten mit einem Teilchenstrahl, welches im Vorfeld einer Bestrahlung durchgeführt wird gemäß Anspruch 9.

Vorrichtungen der hier angesprochenen Art sind bekannt. Sie dienen dazu, bei der Bestrahlung von Tumorgewebe eines Patienten Bewegungen des bestrahlten Zielgebiets auszugleichen. Eine Bewegung des zu bestrahlenden Tumorgewebes tritt insbesondere bei Atmungsbewegungen des Patienten auf. Um den Einfluss von Atembewegungen auf die Dosisdeposition im Tumor zu minimieren, wird meist eine Vergrößerung des Zielvolumens vorgenommen, was zu Belastungen des Patienten führen kann, weil auch gesundes Gewebe bestrahlt wird. Dies erhöht die Wahrscheinlichkeit von Nebenwirkungen. Es existieren bereits Vorschläge zur Reduktion der Dosisbelastung von gesundem Gewebe. Beispielsweise wird vorgeschlagen, die Bewegungsamplitude des zu Bestrahlenden Tumorgewebes durch Kompression der Bauchdecke zu verringern, wodurch die Mobilität des Zwerchfells des Patienten eingeschränkt wird. Auch ist es möglich, die natürliche Atmung durch eine hochfrequente künstliche Atmung (Jet-Ventilation) zu ersetzen. Schließlich kann auch eine hyperoxische Beatmung ins Auge gefasst werden, bei der die Atmung eine Zeit lang stillgelegt werden kann.

Eine weitere Möglichkeit ist die unterbrochene Bestrahlung (Gating), bei der der Patient normal atmet und der Tumor nur während eines bestimmten Zeitfensters bestrahlt wird, innerhalb dessen die gewünschte Position des zu bestrahlenden Tumorgewebes vorliegt. Diese Vorgehensweise führt dazu, dass die Bestrahlungsdauer nachhaltig erhöht wird.

Denkbar ist es auch, die Bestrahlungsdosis nicht auf einmal zu applizieren, sondern während einer Anzahl von Zyklen. Diese mehrfache Bestrahlung (rescanning) erfordert eine Vergrößerung des Zielvolumens. Die Vergrößerung des Zielvolumens ist notwendig, da der Tumor in allen Bewegungszuständen bestrahlt werden muss. Dies kann zu einer Belastung des Patienten führen, weil auch gesundes Gewebe bestrahlt wird. Es werden dabei Interferenzen, wie unten beschrieben, ausgemittelt, um die gewünschte Dosisbelegung des Tumors zu erzielen. Bei diesem Verfahren muss der Tumor in allen Bewegungszuständen getroffen werden, so dass es zu unnötiger Belastung von gesundem Gewebe kommt.

Es hat sich herausgestellt, dass sich ohne eine Nachführung des Teilchenstrahls bei einer Bewegung des zu bestrahlenden Tumorgewebes im Vergleich zu statischer Bestrahlung Unterschiede ergeben, die das Bestrahlungsergebnis beeinträchtigen. Es ergeben sich in dem zu bestrahlenden Gewebe Bereiche, die nicht bzw. mehrfach bestrahlt werden, da sich Bestrahlungsfortschritt und Tumorbewegung überlagern.

Bekannt ist schließlich auch eine Vorrichtung, die zur Bestrahlung eines Tumorgewebes herangezogen wird und bei der eine Steuereinrichtung vorgesehen ist, mit deren Hilfe eine Lageveränderung des Tumorgewebes während der Bestrahlung ausgeglichen werden kann (DE 10 31 071 A1). Dabei ist es erforderlich, den genauen Verlauf der Bestrahlung in zeitlicher Korrelation zur Tumorbewegung zu erfassen, um die applizierte Dosis nachzuvollziehen.

Des weiteren ist aus der DE 100 31 074 A1 eine Vorrichtung gemäß des Oberbegriffs von Anspruch 1 bekannt.

Darüber hinaus beschreibt Paul Keall in 4-Dimensional Computed Tomography Imaging and Treatment Planning (erschienen in Seminars in Radiation Oncology, Vol 14, No 1 (January), 2004: pp 81-90)ein Verfahren zur Dosisberechnung welches die Erfassung der verschiedenen Positionen des Tumorgewebes während eines Atemzyklus beinhaltet.

Aufgabe der Erfindung ist es daher, eine Vorrichtung zu schaffen, die es ermöglicht, eine präzise zeitliche Korrelation zwischen Bestrahlungsabfolge und Tumorbewegung zu realisieren.

Zur Lösung dieser Aufgabe wird eine Vorrichtung vorgeschlagen, die die in Anspruch 1 genannten Merkmale aufweist. Die Vorrichtung umfasst die üblichen Elemente, nämlich einen Beschleuniger zur Erzeugung eines als Behandlungsstrahl bezeichneten Teilchenstrahls mit vorgegebener Teilchenenergie jeweils pro Scheibe, einer Rasterscaneinrichtung, die den Teilchenstrahl in zwei aufeinander senkrecht stehenden Richtungen ablenkt und scheibenweises Abtasten des Tumorgewebes ermöglicht. Die Vorrichtung weist außerdem einen Modulator auf, der die Energie des Teilchenstrahls verändern kann, sodass der durch die Beschleunigungsenergie vorgegebene Bereich maximaler Dosisbelegung, das sogenannte Bragg-Maximum eines Teilchenstrahls, das in unterschiedlichen Tiefen des Tumorgewebes auftritt, schnell verändert werden kann, um so die Eindringtiefe an Änderungen der Anatomie anzupassen. Eine Erfassungseinrichtung der Vorrichtung dient dazu, die Position des Tumorgewebes und dessen Verlagerungen zu erfassen. Sie ermöglicht also ein zeitlich aufgelöstes Erfassen der Position des Tumorgewebes. Die mittels der Erfassungseinrichtung gewonnen Daten werden einer ersten Speichereinrichtung zugeführt. Darüber hinaus kann die Bewegung des Tumors, beispielsweise Atumungsbewegung eines Patienten, wie vor dem Bestrahlungsvorgang aufgenommen, in dem Speicher abgelegt werden. Die Bewegung des Tumors, beispielsweise Atembewegungen eines Patienten, wird vor einem Bestrahlungsvorgang aufgenommen und in dem Speicher abgelegt. Damit ist es möglich, den Teilchenstrahl während eines Bestrahlungsvorgangs so abzulenken, dass er der Bewegung des Tumorgewebes im Wesentlichen folgt und die geforderte Eindringtiefe jeweils über den Modulator angepasst wird.

Die erfindungsgemäße Vorrichtung zeichnet sich dadurch aus, dass ein Modul vorgesehen ist, das einerseits die Daten des Bestrahlungsverlaufs aufzeichnet. Es werden also die durch die Rasterscaneinrichtung vorgegebenen Positionen des Teilchenstrahls über der Zeit erfasst, außerdem die mittels des Beschleunigers für die Bestrahlung bereitgestellte Energie des Teilchenstahls, inklusive einer möglichen Modulation. Damit kann bestimmt werden, in welcher Tiefe das Bragg-Maximum eines Teilchenstrahls liegt.

Andererseits erfasst das Modul Daten einer während der Bestrahlung eines Patienten eingesetzten Erfassungseinrichtung, die über der Zeit die Position des zu bestrahlenden Tumorgewebes erfasst. Dabei ist es möglich, die Position mittels einer Kamera indirekt zu ermitteln, beispielsweise durch auf die Haut des zu bestrahlenden Patienten aufgebrachte Farbmarkierungen oder Lichtquellen, insbesondere Leuchtdioden deren Bewegung während eines Bestrahlungsvorgangs erfasst wird. Weitere Möglichkeiten könnten direkte fluoroskopische Erfassung der Tumorposition oder Erfassung der Oberflächenbewegung des Patienten sein.

Die Vorrichtung zeichnet sich also dadurch aus, dass bei einem Bestrahlungsvorgang exakte Informationen über den Bestrahlungsverlauf und über die Bewegung des zu bestrahlenden Tumorgewebes vorliegen. Der Zusammenhang zwischen Bestrahlungsverlauf und Bewegung des Tumorgewebes wird durch eine Korrelationseinheit innerhalb des Moduls erfasst. Die Daten der Korrelationseinheit geben Aufschluss darüber, welche Dosierung welchem Tumorbereich beziehungsweise Rasterpunkt zuzuordnen ist.

Weitere Ausgestaltungen ergeben sich aus den Unteransprüchen.

Des weiteren wird ein Verfahren zur Dosisberechnung für gescannte Teilchenstrahlen für eine Vorrichtung zur Bestrahlung von Tumorgewebe eines Patienten mit einem Teilchenstrahl, welches im Vorfeld einer Bestrahlung durchgeführt wird, vorgeschlagen.

Die Erfindung wird im Folgenden anhand der Zeichnung näher erläutert. Es zeigen:
- Figur 1: eine Prinzipskizze einer Vorrichtung zum Bestrahlen von Tumorgewebe eines Patienten und
- Figur 2: eine Prinzipskizze eines Teils eines Tumors zur Erläuterung des Einflusses einer Bewegung auf einen Bestrahlungsvorgang.

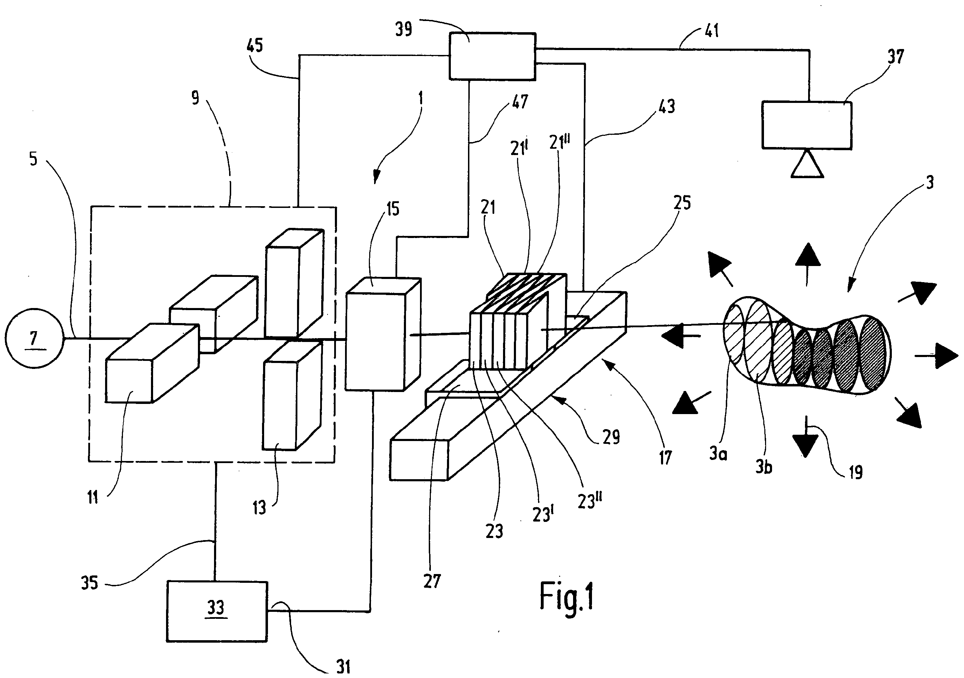

Figur 1 zeigt eine Prinzipskizze einer Vorrichtung 1 zum scheibenweisen Bestrahlen von Tumorgewebe 3 mittels eines Teilchenstrahls 5, der von einem hier nur angedeuteten Beschleuniger 7 zur Verfügung gestellt wird. Der Beschleuniger liefert die Teilchen mit der Energie, die für die aktuell zu bestrahlende Schicht erforderlich ist. Der Teilchenstrahl 5 wird mittels einer Rasterscaneinrichtung 9 sowohl in horizontaler Richtung als auch in vertikaler Richtung abgelenkt, um das Tumorgewebe 3 scheibenweise abzutasten. Dazu ist die Rasterscaneinrichtung 9 hier beispielhaft mit einem ersten Magnetpaar 11 und einem zweiten Magnetpaar 13 versehen. Die zu bestrahlende Scheibe wird durch die vom Beschleuniger gelieferte Teilchenenergie angesteuert. Als Teilchen werden vorzugsweise ¹²C-Teilchen verwendet.

Der die Rasterscanneinrichtung 9 verlassende Strahl verläuft durch einen Teilchenzähler 15, der beispielsweise als Ionisationskammer ausgebildet sein kann, tritt dann durch einen strahlabwärts angeordneten Modulator 17 und gelangt dann zu dem zu bestrahlenden Tumorgewebe 3. Dieses kann sich, wie durch Pfeile 19 angedeutet, bewegen.

Der Modulator 17 kann mindestens zwei einander gegenüberliegende, keilförmig ausgebildete Modulatorplatten 21, 23 aufweisen, die mittels eines geeigneten Antriebs aufeinander zu- und voneinander wegbewegt werden können, sodass der Teilchenstrahl 5 durch ein mehr oder weniger dickes Modulatormaterial hindurchtritt, bevor er auf das Tumorgewebe 3 trifft. Vorzugsweise weist der Modulator 17 eine Anzahl von mehreren nebeneinander liegenden Platten 21, 21', 21" usw. sowie nebeneinanderliegende Modulatorplatten 23, 23', 23" usw. auf, wobei jeweils die nebeneinanderliegenden Platten 21, 21' usw. einen ersten Antrieb 25 und die nebeneinanderliegenden Platten 23, 23' usw. einem zweiten Antrieb 27 zugeordnet sind, die Teil einer Antriebseinrichtung 29 sind. Modulatoren dieser Art sind bekannt, sodass hier nicht näher auf diese eingegangen wird.

Mittels des Modulators 17 wird die Energie des Teilchenstrahls 5 moduliert, damit wird die in Richtung des Teilchenstrahls gemessene Position des Bragg-Maximums variiert, insbesondere wird damit die Eindringtiefe so variiert, dass sie auch bei beispielsweise Atmungsbewegungen und den daraus resultierenden Dichteveränderungen im Patienten in der gewünschten Schicht bleibt.

Mit Hilfe der Rasterscaneinrichtung 9 wird also das Tumorgewebe 3 scheibenweise abgetastet beziehungsweise mit Teilchen des Teilchenstrahls beaufschlagt. Der Modulator 17 dient dazu, die in Richtung des Teilchenstrahls 5 gemessene Position einer Abtastscheibe an den Zustand der Atembewegung anzupassen.

In Figur 1 sind innerhalb des Tumorgewebes 3 eine Anzahl von Scheiben 3a, 3b usw. angedeutet.

Die in einem Bereich des Tumors deponierte Dosis des Teilchenstrahls 5 hängt von der Anzahl der im Teilchenstrahl 5 vorhandenen Teilchen ab. Während eines Bestrahlungsvorgangs wird mittels des Teilchenzählers 15 die auf das Tumorgewebe 3 einwirkende Teilchenzahl ermittelt. Bei Erreichen der gewünschten Teilchenzahl wird über eine Leitung 31 ein Signal an eine Steuereinrichtung 33 abgegeben, die über eine Leitung 35 mit der Rasterscanneinrichtung 9 verbunden ist. Wenn mittels des Teilchenzählers 15 die gewünschte Teilchenzahl erfasst wird, erfolgt eine Aktivierung der Rasterscanneinrichtung 9 über die Leitung 35 dergestalt, dass der nächste Rasterpunkt innerhalb des Tumorgewebes 3 angesteuert wird.

Die Vorrichtung 1 umfasst weiterhin eine Erfassungseinrichtung 37, außerdem ein Modul 39, das über eine Leitung 41 mit der Erfassungseinrichtung 37, über eine Leitung 43 mit dem Modulator 17 und über eine Leitung 45 mit der Rasterscanneinrichtung 9 verbunden ist. Schließlich ist das Modul 39 über eine Leitung 47 mit dem Teilchenzähler 15 verbunden.

Besonders bevorzugt wird eine Vorrichtung 1, die zur Erfassung des Bewegungsablaufs eines Tumorgewebes, beispielsweise während der Atmung des Patienten, eine Erfassungsvorrichtung aufweist, die als Fluoroskopiesystem oder Kamerasystem ausgebildet ist.

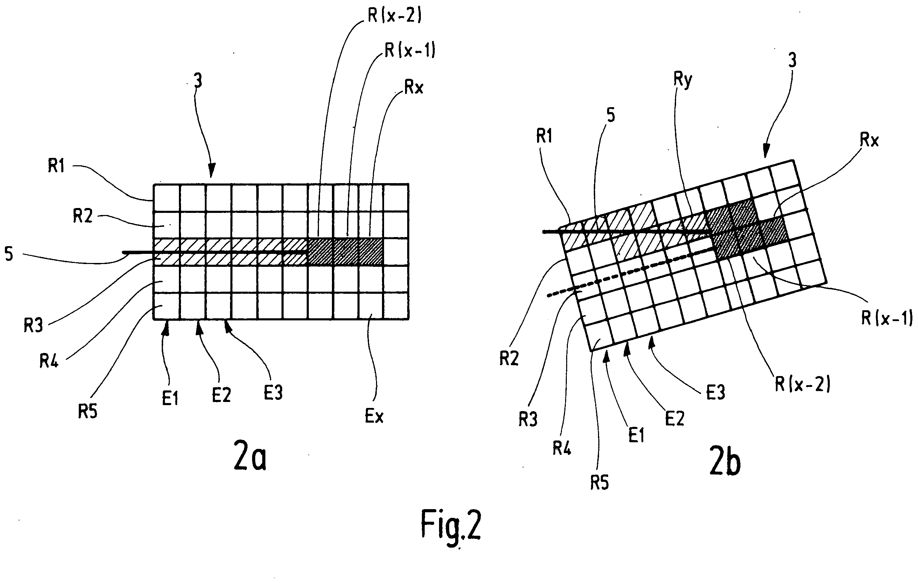

Figur 2 zeigt eine Prinzipskizze eines Teils des Tumorgewebes 3 und zwar links gemäß Ziffer 2a in einer ersten Position und rechts gemäß Ziffer 2b in einer beispielsweise aufgrund von Atmungsbewegungen zweiten verkippten Position.

Durch Quadrate wird angedeutet, dass das Tumorgewebe 3 rasterförmig bestrahlt wird, wobei in Figur 2a Ebenen E1, E2 und E3 usw. dargestellt sind. Innerhalb einer Ebene vorhandene Quadrate sollen einzelne Rasterpunkte andeuten. Der Ausschnitt des Tumorgewebes in Figur 2a zeigt die Rasterpunkte R1 bis R5.

Aus Figur 2a ist ersichtlich, dass mittels des Teilchenstrahls 5 ein Rasterpunkt Rx in der Ebene Ex mit einer Dosis beaufschlagt wird. Links von dem Rasterpunkt Rx liegende Rasterpunke R(x-1), R(x-2) usw. bis R3 werden dabei bereits mit einer Teil-Dosis beaufschlagt. An einem Rasterpunkt tragen also nicht nur die jeweils dort gelegenen Bragg-Maxima zur Dosisdeposition bei, sondern auch Strahlen, deren Bragg-Maxima tiefer im Gewebe liegen.

Figur 2b zeigt das Teilelement des Tumorgewebes 3 in einer gegenüber Figur 2a gegen den Uhrzeigersinn verdrehten Position. Gleiche Bezugsziffern dienen dazu, gleiche Teile zu kennzeichnen, die bereits in Figur 2a erläutert wurden. Es wird also deutlich, dass der hier dargestellte Teil des Tumorgewebes 3 in Ebenen E1, E2, E3 usw. angeordnete Rasterpunkte aufweist, wobei in der Ebene E1 die Rasterpunkte R1 bis R5 liegen.

Durch Modulation der Strahllage trifft der Teilchenstrahl 5 hier wiederum auf den Rasterpunkt Rx. Da das Tumorgewebe 3 verkippt ist, durchläuft der Teilchenstrahl 5, anders als in Figur 2a, nicht einer Reihe nebeneinanderliegender Rasterpunkte sondern trifft auf Rasterpunkte, die in unterschiedlichen Reihen liegen. Dabei werden in der obersten Reihe des Ausschnittes der Tumorgewebes 3 vier nebeneinanderliegende Rasterpunkte getroffen, nämlich der Rasterpunkt R1 und drei rechts danebenliegende Rasterpunkte. In der zweiten Reihe werden in einem Abstand zum Rasterpunkt R2 liegende Rasterpunkte getroffen und schließlich Rasterpunkte, die in der gleichen Reihe liegen wie der in der Ebene E1 liegender Rasterpunkt E3.

Der Rasterpunkt Ry wird mit einer etwas geringeren Dosis beaufschlagt. Die links davon liegenden Rasterpunkte werden wiederum mit einer geringeren Dosis bestrahlt.

Aus den Figuren 2a und 2b ist ersichtlich, dass bei der Bestrahlung des Rasterpunkts Rx mittels eines Teilchenstrahls, wobei das Bragg-Maximum des Teilchenstrahls in Rx liegt, unterschiedlich angeordnete Rasterpunkte bereits mit einer Dosierung beaufschlagt werden. Die unterschiedliche relative Lage der Rasterpunkte zueinander wird durch die Tumorbewegung hervorgerufen, wie hier dargestellt zum Beispiel durch Rotationsanteile.

Im Folgenden wird auf die Funktion der in Figur 1 dargestellten Vorrichtung zum Bestrahlen von Tumorgewebe eines Patienten und auch das nicht-beanspruchte Verfahren zur Kompensation von Bewegungen eines Patienten beziehungsweise des bestrahlten Bereichs eines Patienten während einer Bestrahlung mit einem Teilchenstrahl mittels einer Vorrichtung gemäß Figur 1 näher eingegangen.

Das Tumorgewebe 3 eines Patienten wird mittels einer Erfassungseinrichtung, beispielsweise der Erfassungseinrichtung 37, während einer Bewegung, beispielsweise beim Atmen des Patienten, erfasst. Die Erfassung der verschiedenen Positionen des Tumorgewebes 3 während eines Bewegungsablaufs kann auch dergestalt erfolgen, dass die Bewegung in quasi-statische, vorzugsweise nicht überlappende Bewegungsphasen zerlegt wird. Es findet also eine zeitlich aufgelöste Bewegungserfassung des Tumorgewebes statt.

Vor der Bestrahlung erfolgt die Bewegungserfassung mittels einer Einrichtung, die vorzugsweise als 4D-CT (4D Computertomograph) ausgelegt ist. Die Erfassung der dabei erzeugten Bewegungsphasen kann indirekt erfolgen, beispielsweise mittels einer Videokamera, die Farbpunkte auf der Haut eines Patienten oder dort angebrachte Lichtquellen erfasst. Während der Bestrahlung können die vorliegenden Bewegungszustände dann zum Beispiel den Bewegungsphasen des 4D-CT zugeordnet werden. Vorzugsweise erfolgt die Bewegungserfassung allerdings mittels einer Erfassungseinrichtung, die als Fluoroskopiesystem oder Kamerasystem zur Oberflächendetektion ausgelegt ist. Es ist also möglich, die örtliche Position des Tumorgewebes während eines Bewegungsablaufes kontinuierlich oder zu bestimmten Zeitpunkten zu bestimmen.

Diese Daten der verschiedenen Bewegungsphasen werden in einer in Figur 1 nicht dargestellten ersten Speichereinrichtung abgelegt.

Während eines Bestrahlungsvorgangs wird die Bewegung des Tumors durch das Erfassungssystem den Bewegungsphasen zugeordnet. Eine Korrelation in einem Modul 39 mit den Daten aus der ersten Speichereinrichtung wird dazu verwendet, die Rasterscaneinrichtung 9 und den Modulator 17 so zu steuern, dass der Teilchenstrahl 7 das Tumorgewebe 3 in den verschiedenen Bewegungsphasen wie geplant bestrahlt.

Diese Daten werden also dazu verwendet, die Rasterscaneinrichtung 9 so zu steuern, dass eine scheibenweise Abtastung des Tumorgewebes 3 auch unter Bewegung erfolgt.

Die Eindringtiefe des Teilchenstrahls 5 in das Tumorgewebe, also die Ebene 3a, 3b usw. innerhalb des Tumorgewebes 3, in der das Bragg-Maximum des Teilchenstrahls liegt, wird durch Aktivierung des Modulators 17 festgelegt, dessen Modulatorplatten 21, 21', 21" und 23, 23', 23" usw. mittels der Antriebe 25 und 27 mehr oder weniger weit aufeinander zu bewegt werden.

Bei der Bestrahlung eines Rasterpunkts innerhalb des Tumorgewebes 3 wird die Teilchenzahl des Teilchenstrahls 5 mittels des beispielsweise als Ionisationskammer ausgebildeten Teilchenzählers 15 ermittelt. Wenn die gewünschte Teilchenzahl an einem Rasterpunkt erreicht ist, wird die Rasterscanneinrichtung 9 so aktiviert, dass der nächste Rasterpunkt bestrahlt wird. Während eines Bestrahlungsvorgangs wird die aktuelle Position des Patienten bzw. des Tumorgewebes 3 mit Hilfe der Erfassungseinrichtung 37 zeitlich aufgelöst erfasst. Dabei kann hier eine indirekte Erfassung beispielsweise über eine Videokamera oder eine direkte Erfassung beispielsweise über ein Fluoroskopiesystem oder Kamerasystem erfolgen.

Das Modul 39 erfasst einerseits den laufenden Bestrahlungsvorgang, indem über der Zeit der aktuelle Rasterpunkt innerhalb des Tumorgewebes 3 und die dort eingestrahlte Dosierung erfasst werden. Auf diese Weise ist exakt bekannt, welcher der Rasterpunkte des Tumorgewebes 3 mit welcher Dosis zu welchem Zeitpunkt bestrahlt wurde. Umgekehrt kann damit auch die pro Rasterpunkt eingestrahlte Dosierung, die durch den Bestrahlungsvorgang erreicht wurde, festgestellt werden.

Andererseits wird mit Hilfe des Moduls 39 während der Bestrahlung die Bewegung des Tumorgewebes 3 ermittelt. Dadurch ist bekannt, welcher tatsächliche Rasterpunkt innerhalb des Körpers des Patienten mit welcher Dosierung in einem bestimmten Zeitpunkt beaufschlagt wurde.

Die Daten bezüglich des Bestrahlungsvorgangs und die der während des Bestrahlungsvorgangs erfolgenden Bewegung des Tumorgewebes 3 werden miteinander korreliert, um die genannte Aussage über die Dosis an einem Rasterpunkt im Körper der Patienten festlegen zu können.

Die durch die Korrelation gewonnenen Informationen können in einer zweiten Speichereinrichtung, die hier nicht dargestellt ist, abgespeichert werden. Bei entsprechender Auslegung der ersten Speichereinrichtung können diese Daten auch dort abgelegt werden. Sie liefern ein Protokoll einer Bestrahlung, das nach der Bestrahlung ausgewertet werden kann. Dadurch wird man in die Lage versetzt, den Bestrahlungserfolg zu beurteilen. Beispielsweise kann festgestellt werden, ob und welche Rasterpunkte eines Tumors mit einer zu geringen Dosis bestrahlt wurden.

In der Regel werden zur Behandlung von Tumorgewebe mehrere Behandlungsabschnitte, die auch als Fraktionen bezeichnet werden, vorgesehen. Aufgrund der in dem Protokoll niedergelegten Daten kann die bei einer nachfolgenden Bestrahlung für die Rasterpunkte des Tumorgewebes zu wählende Dosis neu bestimmt werden.

Besonders bevorzugt werden die durch die Korrelation der Daten des Moduls 39 gewonnenen Informationen dazu verwendet, in einen laufenden Bestrahlungsvorgang einzugreifen: Es ist damit möglich, eine Dosisanpassung für mittels einer Rasterscaneinrichtung 9 auf ein Tumorgewebe 3 gerichtete Teilchenstrahlen 5 bei einem bewegten Zielgebiet durchzuführen. Bei einem Bestrahlungsvorgang werden die einzelnen Rasterpunkte des Tumorgewebes mit einem Teilchenstrahl 5 beaufschlagt. Vor dem Bestrahlen eines weiteren Rasterpunkts kann anhand der in dem Modul 39 vorhandenen Daten bestimmt werden, mit welcher Dosis der nächste Rasterpunkt zu beaufschlagen ist. Dabei kann berücksichtigt werden, ob dieser Rasterpunkt bei der Bestrahlung vorangegangener Rasterpunkte möglicherweise bereits mit einer Teil-Dosis beaufschlagt wurde (siehe Figur 2). In diesem Fall wird dann die Strahlungsdosis für diesen vorbestrahlten Rasterpunkt angepasst. Dabei kann die Anpassung zu einer Erhöhung oder aber auch Reduktion der zu applizierenden Dosis führen.

Bei einer derartigen Ausgestaltung der Vorrichtung 1 werden also die in dem Modul 39 vorhandenen Daten bezüglich des Bestrahlungsvorgangs und die Daten bezüglich der Bewegung des Patienten korreliert und die daraus gewonnenen Informationen unmittelbar zur Beeinflussung der Rasterscanneinrichtung 9 und des Modulators 17 herangezogen.

Vorzugsweise werden vor einem Bestrahlungsvorgang mögliche Bewegungsphasen des Tumorgewebes erfasst und abgespeichert. Vor der Einleitung des Teilchenstrahls 5 in einen bestimmten Rasterpunkt des Tumorgewebes kann dann anhand eines Korrekturwertes, der aus einem Speicher abgerufen wird, die erforderliche Korrektur der Strahllage sowie Stellung des Modulators und auch die Dosierung für den nächsten Rasterpunkt bestimmt werden.

Durch die Verbindung des Moduls 39 mit der Rasterscaneinrichtung 9 und dem Modulator 17 wird hier ein Regelkreis gebildet, der das nicht-beanspruchte Verfahren zum Bestrahlen von Tumorgewebe eines Patienten mit einem Teilchenstrahl wesentlich verbessert, weil Bewegungen des Tumorgewebes während einer Bestrahlung kompensiert werden.

Im Vorfeld der Bestrahlung von Tumorgewebe 3 eines Patienten mittels der hier beschriebenen Vorrichtung 1 werden Verfahren zur Dosisberechnung und zur Bestrahlungsplanung durchgeführt.

Bei dem Verfahren zur Dosisberechnung für die gescannten Teilchenstrahlen werden bei einem Patienten die verschiedenen Positionen des zu bestrahlenden Tumorgewebes erfasst. Es wird also der Bewegungsablauf des Tumorgewebes 3 ermittelt, um später bei einem Patienten einen Tumor bestrahlen zu können, der nicht statisch ist, sondern der sich während des Bestrahlungsvorgangs, beispielsweise aufgrund der Atmung des Patienten, bewegt.

Die Bewegung des Tumorgewebes wird in quasi-statische Bewegungsphasen aufgeteilt, die sich vorzugsweise nicht überlappen. Die Bewegung wird also nicht kontinuierlich, sondern bezüglich einzelner separater Bewegungsphasen abgespeichert.

Für jede Bewegungsphase wird ein Teil-Bestrahlungsplan erstellt, wobei die Dosis für eine Bewegungsphase, während derer das Tumorgewebe 3 mit einem Teilchenstrahl 5 abgescannt wird, aus dem Teilbestrahlungsplan und der zugehörigen Bewegungsphase berechnet wird. Man erhält damit für jede Bewegungsphase sowohl einen Teil-Bestrahlungsplan als auch eine Teil-Dosis.

Die anatomischen Strukturen in den verschiedenen Bewegungsphasen kommen aufgrund der Bewegung nicht immer an den exakt gleichen Stellen zu liegen. Damit können die Einzeldosen pro Bewegungsphase, also die Teil-Dosen nicht ohne weiteres summiert werden. Es ist erforderlich, die den Bewegungsphasen zugeordneten Teil-Dosen auf einen Referenzzustand unter Berücksichtigung der anatomischen Bewegungen zu transformieren.

Erst nach dieser Transformation ist eine Aufsummierung der Teil-Dosen zu einer Gesamtdosis möglich.

Um während einer Bestrahlung die Position des Bragg-Maximums definieren zu können, muss dieses aus dem Referenzzustand mittels eines Korrekturwerts in andere Phasen transformiert werden. Dieser wird von einer hier nicht dargestellten Rechenkomponente bereitgestellt.

Dabei ist ein erster Korrekturwert zu berücksichtigen, der aus der Bewegung des Tumorgewebes 3 resultiert. In diesem Fall wird als Korrekturwert ein Vektor oder ein Korrektur-Tripel verwendet, bestehend aus zwei lateralen Korrekturwerten zur Strahlnachführung und einem Tiefenkorrekturwert, aus dem die erforderliche Energiemodulation hervorgeht. Außerdem muss bei der Bestimmung des Korrekturwerts berücksichtigt werden, dass einzelne Rasterpunkte des Tumorgewebes 3 bei der Bestrahlung eines anderen Rasterpunkts bereits mit einer Teil-Dosis belegt werden. Dieser Dosisbeitrag ist je nach Ablauf der Bestrahlung unterschiedlich. Als Korrekturwert muss für diesen Fall eine Vorbestrahlungsvariable herangezogen werden.

Mittels des hier beschriebenen Verfahrens ist es möglich, die dem Referenzzustand zuzuordnenden Parameter für den Teilchenstrahl abzuspeichern. Zusätzlich wird eine Wertetabelle in einem Speicher abgelegt, die die Korrekturwerte einerseits bezüglich der Bewegung und andererseits bezüglich der Vorbestrahlung enthält.

Das der eigentlichen Bestrahlung ebenfalls vorgeschaltete Verfahren zur Bestrahlungsplanung für gescannte Teilchenstrahlen für eine Vorrichtung 1 zum Bestrahlen von Tumorgewebe 3 eines Patienten mit einem Teilchenstrahl 5 umfasst die folgenden Schritte:
Wie bereits bei dem Verfahren zur Dosisberechnung werden verschiedene Positionen des Tumorgewebes eines Patienten während eines Bewegungsablaufs, also beispielsweise während der Atmung des Patienten, erfasst. Dabei wird die Bewegung in quasi-statische, vorzugsweise nicht überlappende Bewegungsphasen aufgeteilt.

In einem weiteren Schritt wird die anatomische Lage des aktuellen Rasterpunkts für eine geplante Bestrahlung aus der Kombination eines Rasterpunkts im Referenzzustand mit einem Bewegungszustand ermittelt, der dem aktuellen Rasterpunkt zugeordnet ist. Es wird also für jede Kombination aus Rasterpunkt und Bewegungszustand die anatomische Lage des aktuellen Rasterpunkts anhand der oben erwähnten Transformationen verfolgt.

Für eine Abweichung des aktuellen Rasterpunkts gegenüber einem anhand des Referenzzustandes bestimmten Rasterpunkts wird für den aktuellen Rasterpunkt ein Korrekturwert berechnet. Dieser Korrekturwert wird als Vektor oder als Korrektur-Tripel bezeichnet.

Diese Korrekturwerte für jeden Rasterpunkt werden kombiniert mit jedem Bewegungszustand in Form einer Tabelle in einem Speicher abgelegt. Auch für die Korrekturwertsätze werden für jeden Rasterpunkt die mit den Bewegungszuständen möglichen Kombinationen in einem Speicher abgelegt. Damit können die Korrekturwerte bei einer Bestrahlung anhand des hier ermittelten Bestrahlungsplans anhand der dann beobachteten Bewegung des Tumorgewebes angewendet werden.

Für jeden Rasterpunkt kann die Dosisdeposition von anderen Rasterpunkten berücksichtigt werden, indem alle Kombinationen von Bewegungsphasen und Rasterpunktzuordnungen berücksichtigt werden. Daraus kann dann ein Satz Vorbestrahlungsvariablen für alle möglichen Bestrahlungsverläufe bestimmt werden. Bei dem Verfahren zur Bestrahlungsplanung kann, wie auch bei dem Verfahren zur Dosisberechnung, die anatomische Lage des aktuellen Rasterpunkts mittels eines 4D-CT's oder einer 4D-MR-Einrichtung (4D Magnetresonanz-Einrichtung) bestimmt werden. Damit liegen die anatomischen Daten als zeitaufgelöste Daten vor.

Auch hier ist, wie oben anhand des Verfahrens zur Dosisberechnung beschrieben, eine Transformation der Teil-Dosen auf einen Referenzzustand erforderlich, bevor die Teil-Dosen aufsummiert werden können.

## Patentansprüche

1. Vorrichtung zum scheibenweisen Bestrahlen von Tumorgewebe (3) eines Patienten mit einem Teilchenstrahl, mit
- einem Beschleuniger (7) zur Erzeugung eines Teilchenstrahls (5) mit vorgegebener Energie jeweils pro Scheibe,
- einer auf den Teilchenstrahl (5) einwirkenden Rasterscaneinrichtung (9) zum scheibenweisen Abtasten des Tumorgewebes (3),
- einem Modulator (17) zum Modulieren der Energie des Teilchenstrahls (5),
- einer Erfassungseinrichtung (37) zum zeitlich aufgelösten Erfassen der Position des Tumorgewebes (3) und mit
- einer ersten Speichereinrichtung zum Ablegen von Daten bezüglich des Tumorgewebes (3), die vor einem Bestrahlungsvorgang ermittelt wurden, und zur Abgabe von diesen Daten an die Rasterscanvorrichtung (9) und an den Modulator (17) und
- einem Modul (39), das eine pro Rasterpunkt mit dem Teilchenstrahl eingestrahlte Dosierung und die Daten der Erfassungseinrichtung (37) während eines Bestrahlungsvorgangs erfasst
**gekennzeichnet durch**
- eine Korrelationseinheit, die die pro Rasterpunkt mit dem Teilchenstrahl eingestrahlte Dosierung und die Daten der Erfassungseinrichtung (37) in zeitliche Beziehung zueinander setzt, so dass einem Tumorbereich eine applizierte Dosierung zugeordnet werden kann.

2. Vorrichtung nach Anspruch 1, **dadurch gekennzeichnet, dass** eine zweite Speichereinrichtung vorgesehen ist, welche die von dem Modul (39) erfassten Daten speichert.

3. Vorrichtung nach Anspruch 1 oder 2, **gekennzeichnet durch** einen Teilchenzähler (15), der der Rasterscaneinrichtung (9) zugeordnet ist.

4. Vorrichtung nach einem der vorhergehenden Ansprüche, **dadurch gekennzeichnet, dass** das Modul (39) mit der Rasterscaneinrichtung (9) und/oder dem Modulator (17) zur Ausbildung eines Regelkreises verbunden ist.

5. Vorrichtung nach einem der vorhergehenden Ansprüche, **dadurch gekennzeichnet, dass** die erste Speichereinrichtung Daten bezüglich verschiedener Tumorpositionen umfasst.

6. Vorrichtung nach einem der vorhergehenden Ansprüche, **gekennzeichnet durch** eine Vergleichseinrichtung, die den aktuellen Bestrahlungsverlauf mit gespeicherten Strahlungsdaten vergleicht.

7. Vorrichtung nach einem der vorhergehenden Ansprüche, **gekennzeichnet durch** eine Rechenkomponente, die einen Korrekturwert bereitstellt.

8. Vorrichtung nach einem der vorhergehenden Ansprüche, **dadurch gekennzeichnet, dass** die Erfassungsvorrichtung als Fluoroskopie- oder Kamerasystem ausgebildet ist.

9. Verfahren zur Dosisberechnung für gescannte Teilchenstrahlen für eine Vorrichtung zur Bestrahlung von Tumorgewebe eines Patienten mit einem Teilchenstrahl, welches im Vorfeld einer Bestrahlung durchgeführt wird, mit folgenden Schritten:
- Erfassung der verschiedenen Positionen des Tumorgewebes während eines Bewegungsablaufs,
- Aufteilung der Bewegung in quasistatische, vorzugsweise sich nicht überlappende, Bewegungsphasen,
- Erstellung eines Teil-Bestrahlungsplans eines Referenzbestrahlungsplans pro Bewegungsphase, wobei die Dosis für eine Bewegungsphase aus dem Teil-Bestrahlungsplan und der zugehörigen Bewegungsphase berechnet wird,
- Transformation der den Bewegungsphasen zugeordneten Teildosen entsprechend der Bewegungsphase auf einem Referenzzustand und
- Aufsummierung der Einzeldosen zu einer Gesamtdosis.

10. Verfahren nach Anspruch 9, ferner mit den Schritten:
- Ermittlung der anatomischen Lage eines aktuellen Rasterpunkts für eine geplante Bestrahlung aus der Kombination eines Rasterpunkts im Referenzzustand mit einem Bewegungszustand, der dem aktuellen Rasterpunkt zugeordnet ist,
- Berechnung eines Korrekturwertsatzes für den aktuellen Rasterpunkt und
- Abspeichern des Korrekturwertsatzes gemäß des Bewegungszustands in einer in einem Speicher abgelegten Tabelle.

11. Verfahren nach Anspruch 10, **dadurch gekennzeichnet, dass** als Korrekturwertsatz ein erstes Korrekturwerttripel berechnet wird, der der Bewegung des zu bestrahlenden Tumorgewebes Rechnung trägt, darüber hinaus ein zweiter Korrekturwert, der die Vorbestrahlung eines Rasterpunkts des Tumorgewebes berücksichtigt.

12. Verfahren nach Anspruch 10 oder 11, **dadurch gekennzeichnet, dass** der zweite Korrekturwertsatz für alle möglichen Kombinationen aus Bewegungszuständen und Bestrahlungsabläufen berechnet wird.

13. Verfahren nach einem der Ansprüche 10 bis 12, **dadurch gekennzeichnet, dass** die anatomische Lage des aktuellen Rasterpunkts mittels eines 4D-CT's oder einer 4D-MR-Einrichtung bestimmt wird.

## Claims

1. A device for slice-by-slice irradiation of tumorous tissue (3) of a patient with a particle beam, comprising:
- an accelerator (7) for generating a particle beam (5) with predetermined energy for each slice;
- a raster scanning device (9) acting on the particle beam (9) for scanning the tumorous tissue slice by slice;
- a modulator (17) for modulating the energy of the particle beam (5);
- a detection device (37) for detecting the location of the tumorous tissue in time-resolved manner, and having
- a first memory device for storing data relating to the tumorous tissue (3), which data were determined prior to an irradiation operation, and for supplying said data to the raster scanning device (9) and to the modulator (17); and
- a module (39) which registers, during an irradiation operation, a dose irradiated by the particle beam per raster point and the data of the detection device (37);
**characterized by**
- a correlation unit which correlates in time the dose irradiated by the particle beam per raster point and the data of the detection device (37), so as to be able to associate an applied dose to a tumour region.

2. The device according to claim 1, **characterized in that** a second memory device is provided, which stores the data registered by said module (39).

3. The device according to claim 1 or 2, **characterized by** a particle counter (15) which is associated with the raster scanning device (9).

4. The device according to any one of the preceding claims, **characterized in that** the module (39) is connected to the raster scanning device (9) and/or to the modulator (17) so as to form a closed-loop control circuit.

5. The device according to any one of the preceding claims, **characterized in that** the first memory device includes data relating to different tumour locations.

6. The device according to any one of the preceding claims, **characterized by** a comparison means which compares the actual irradiation progress with stored irradiation data.

7. The device according to any one of the preceding claims, **characterized by** a computing component which provides a correction value.

8. The device according to any one of the preceding claims, **characterized in that** the detection device is a fluoroscopy system or a camera system.

9. A method for dose calculation for scanned particle beams for a device for irradiation of tumorous tissue of a patient with a particle beam, comprising the steps of:
- detecting different locations of the tumorous tissue during a movement pattern;
- dividing the movement into quasi-static movement phases that preferably do not overlap each other;
- establishing a partial irradiation plan of a reference irradiation plan per movement phase, wherein the dose for a movement phase is calculated from the partial irradiation plan and the associated movement phase;
- converting the partial doses associated with the movement phases to a reference state corresponding to the movement phase; and
- adding up the individual doses to obtain a total dose.

10. The method according to claim 9, further comprising the steps of:
- determining the anatomical location of an actual raster point for a planned irradiation from the combination of a raster point in the reference state with a state of movement associated with the actual raster point;
- calculating a set of correction values for the actual raster point; and
- storing the set of correction values according to the state of movement in a table stored in a memory device.

11. The method according to claim 10, **characterized in that** a first correction value triplet is calculated as the set of correction values, which takes into account the movement of the tumorous tissue to be irradiated, and
moreover a second correction value which takes into account the previous irradiation of a raster point of the tumorous tissue.

12. The method according to claim 10 or 11, **characterized in that** the second set of correction values is calculated for all possible combinations of states of movement and irradiation sequences.

13. The method according to any one of claims 10 to 12, **characterized in that** the anatomical location of the actual raster point is determined by a 4D CT or a 4D MR device.

## Revendications

1. Installation pour irradier par tranches un tissu tumoral (3) chez un patient au moyen d'un faisceau de particules, avec
- un accélérateur (7) pour la production d'un faisceau de particules (5) avec une énergie prédéterminée respectivement par tranche,
- un dispositif de balayage de trame (9) agissant sur le faisceau de particules (5) pour le balayage par tranches du tissu tumoral (3),
- un modulateur (17) pour moduler l'énergie du faisceau de particules (5),
- un dispositif de détection (37) pour détecter en résolution temporelle la position du tissu tumoral (3) et avec
- un premier dispositif de mémoire pour le stockage de données relatives au tissu tumoral (3), qui ont été déterminées avant une opération d'irradiation, et pour l'envoi de ces données au dispositif de balayage de trame (9) et au modulateur (17), et
- un module (39), qui détecte le dosage irradié avec le faisceau de particules par point de trame et les données du dispositif de détection (37) pendant une opération d'irradiation,
**caractérisé par**
- une unité de corrélation, qui met en relation temporelle l'une avec les autres le dosage irradié avec le faisceau de particules par point de trame et les données du dispositif de détection (37), de telle manière qu'un dosage appliqué puisse être associé à une zone tumorale.

2. Installation selon la revendication 1, **caractérisée en ce qu'**il est prévu un deuxième dispositif de mémoire, qui mémorise les données détectées par le module (39).

3. Installation selon la revendication 1 ou 2, **caractérisée par** un compteur de particules (15), qui est associé au dispositif de balayage de trame (9).

4. Installation selon l'une quelconque des revendications précédentes, **caractérisée en ce que** le module (39) est relié au dispositif de balayage de trame (9) et/ou au modulateur (17) pour la formation d'un circuit de réglage.

5. Installation selon l'une quelconque des revendications précédentes, **caractérisée en ce que** le premier dispositif de mémoire comprend des données relatives à différentes positions de tumeurs.

6. Installation selon l'une quelconque des revendications précédentes, **caractérisée par** un dispositif de comparaison, qui compare la courbe d'irradiation actuelle avec des données d'irradiation mémorisées.

7. Installation selon l'une quelconque des revendications précédentes, **caractérisée par** un composant de calcul, qui fournit une valeur de correction.

8. Installation selon l'une quelconque des revendications précédentes, **caractérisée en ce que** le dispositif de détection est constitué par un système de fluoroscopie ou de caméra.

9. Procédé de calcul de dose pour des faisceaux de particules balayés pour une installation d'irradiation de tissu tumoral chez un patient avec un faisceau de particules, qui est effectué en prévision d'une irradiation, comprenant les étapes suivantes:
- détection des différentes positions du tissu tumoral pendant l'exécution d'un mouvement;
- décomposition du mouvement en phases de mouvement quasi statiques, de préférence sans chevauchement,
- établissement d'un plan d'irradiation partielle d'un plan d'irradiation de référence par phase de mouvement, dans lequel on calcule la dose pour une phase de mouvement à partir du plan d'irradiation partielle et de la phase de mouvement correspondante,
- transformation des doses partielles associées aux phases de mouvement selon la phase de mouvement en un état de référence, et
- sommation des doses individuelles en une dose globale.

10. Procédé selon la revendication 9, comprenant en outre les étapes suivantes:
- détermination de la position anatomique du point de trame actuel pour une irradiation planifiée à partir de la combinaison d'un point de trame dans l'état de référence avec un état de mouvement, qui est associé au point de trame actuel,
- calcul d'un ensemble de valeurs de correction pour le point de trame actuel, et
- mémorisation de l'ensemble de valeurs de correction selon l'état de mouvement dans une table stockée dans une mémoire.

11. Procédé selon la revendication 10, **caractérisé en ce que** l'on calcule comme ensemble de valeurs de correction une première triple valeur de correction, qui tient compte du mouvement du tissu tumoral à irradier, et en outre une deuxième valeur de correction, qui tient compte de l'irradiation préalable d'un point de trame du tissu tumoral.

12. Procédé selon la revendication 10 ou 11, **caractérisé en ce que** l'on calcule le deuxième ensemble de valeurs de correction pour toutes les combinaisons possibles à partir d'états de mouvement et d'allures d'irradiation.

13. Procédé selon l'une quelconque des revendications 10 à 12, **caractérisé en ce que** l'on détermine la position anatomique du point de trame actuel au moyen d'un dispositif 4D-CT ou 4D-MR.
